Europäisches Patentamt

European Patent Office

Office européen des brevets

(11) Veröffentlichungsnummer: **0 330 097**

**A2**

## (12) EUROPÄISCHE PATENTANMELDUNG

(21) Anmeldenummer: 89102822.7

(22) Anmeldetag: 18.02.89

(51) Int. Cl.⁴: **C07C 51/41 , A23K 1/00 , C07C 53/126**

(30) Priorität: 26.02.88 DE 3806192

(43) Veröffentlichungstag der Anmeldung:
**30.08.89 Patentblatt 89/35**

(84) Benannte Vertragsstaaten:
**AT BE CH DE ES FR GB IT LI NL SE**

(71) Anmelder: **NEYNABER CHEMIE GmbH**
**Am Wedenberg Postfach 11 20**
**D-2854 Loxstedt(DE)**

(72) Erfinder: **Hirsch, Albrecht, Dr.**
**Walter-Delius-Strasse 65**
**D-2850 Bremerhaven(DE)**
Erfinder: **Fleischer, Erwin**
**Dwarsweg 1**
**D-2858 Schiffdorf(DE)**

(54) **Verfahren zur Herstellung von pulverförmigen basischen Metallseifen.**

(57) Pulverförmige basische Metallseifen einer Zusammensetzung gemäß der Formel (I)

$$(MO)_n . M(RCOO)_2 \qquad (I)$$

in der MO ein Oxid aus der von CaO, ZnO, MgO, BaO und PbO gebildeten Gruppe oder ein gemischtes Oxid aus der von PbO/CaO, CaO/ZnO, BaO/CdO und BaO/ZnO gebildeten Gruppe, M eines der vorgenannten Metalle, $RCOO^-$ der Rest einer Fettsäure mit 8 bis 34 Kohlenstoffatomen und n eine Zahl von 0,2 bis 2 bedeuten, können erhalten werden, indem man pulverförmige Fettsäuren mit pulverförmigen Metalloxiden in Gegenwart von Wasser und/oder einem $C_1$-$C_3$-Alkanol und/oder einer Säure bei Temperaturen zwischen Umgebungstemperatur und 100 °C und gegebenenfalls verringertem Druck umsetzt und während der Umsetzung gebildetes Wasser fortlaufend entfernt, wobei während der Umsetzung das Reaktionsgemisch in Form von diskreten, freifließenden Teilchen vorliegt. Die erhaltenen Metallseifen eignen sich je nach Zusammensetzung als Viehfutterzusatz oder Stabilisator/Gleitmittel-Zusammensetzungen für die Kunststoffindustrie.

## Verfahren zur Herstellung von pulverförmigen basischen Metallseifen

Die Erfindung betrifft ein Verfahren zur Herstellung von pulverförmigen basischen Metallseifen in einer Zusammensetzung gemäß Formel (I)

$$(MO)_n \cdot M(RCOO)_2 \qquad (I)$$

in der MO ein Oxid aus der von CaO, ZnO, MgO, BaO und PbO gebildeten Gruppe oder ein gemischtes Oxid aus der von PbO/CaO, CaO/ZnO, BaO/CdO und BaO/ZnO gebildeten Gruppe, M eines der vorgenannten Metalle, die Gruppe RCOO⁻ der Rest einer Fettsäure mit 8 bis 34 Kohlenstoffatomen oder von Gemischen dieser Fettsäuren und n eine Zahl von 0,2 bis 2, insbesondere von 1 bis 2, bedeutet.

Die vorgenannten Metallseifen, soweit sie ausschließlich Calcium enthalten, eignen sich insbesondere als Zusatz in Mischfutter für Milchkühe, wodurch die Milchleistung und der Milchfettgehalt gesteigert und eine besser streichfähige Butter gewonnen werden kann. Die gemäß dem Verfahren der Erfindung herstellbaren Metallseifen eignen sich im übrigen als Bestandteile von Stabilisator-/Gleitmittelmischungen bei der Verarbeitung von halogenhaltigen Kunststoffen, insbesondere von PVC.

Basische Bleiseifen, insbesondere Bleistearat, wurden bisher über Fällungsverfahren hergestellt. Dieses Verfahren ist jedoch kostenaufwendig und ergibt zudem ein sehr feinpulveriges, stark staubendes Produkt.

Aus der EP-A 0 163 395 und der GB-A 2 113 521 sind Verfahren zur Herstellung von Futtermitteln bekannt, bei denen flüssige bwz. geschmolzene Fettsäuren in Gegenwart von Proteinen und Kohlehydraten mit Calciumoxid bzw. Calciumhydroxid umgesetzt werden. Dieses Verfahren ist apparativ jedoch sehr aufwendig, da das offenbar noch nicht vollständig abreagierte Reaktionsgemisch zum Nachreagieren und Trocknen auf einem endlosen Band oder dergleichen ausgebreitet werden muß. Bei dieser Arbeitsweise dürften die erhaltenen Calciumseifen beim Weglassen der genannten Zusätze wie Proteine und Kohlehydrate nur schwer in die Form freifließender Teilchen zu bringen sein.

Die Erfindung ist auf ein Verfahren der oben genannten Art gerichtet, bei dem grob- bis feinpulverige basische Metallseifen erhältlich sind, die zudem einen sehr viel höheren Schmelzpunkt als die entsprechenden neutralen Metallseifen aufweisen.

Diese Aufgabe wird erfindungsgemäß dadurch gelöst, daß man in einem Reaktor unter mechanischem Vermischen

a) pulverförmige Fettsäuren der Formel RCOOH, in der R wie oben definiert ist, mit pulverförmigen Metalloxiden bzw. Metalloxidgemischen der Formel MO, in der M wie oben definiert ist, oder die entsprechenden pulverförmigen Metallhydroxide bzw. Metallhydroxidgemische der Formel M(OH)₂ in der obigen Formel (I) entsprechenden stöchiometrischen Mengen in Gegenwart von 0,1 bis 5 Gew.-% Wasser und/oder C₁-C₃-Alkanolen und/oder 0,1 bis 1 Gew.-% einer Säure mit einem pKₛ-Wert von 5 oder weniger als 5 bei Temperaturen zwischen Umgebungstemperatur und 100° C und einem Druck, der gleich dem oder geringer als der Umgebungsdruck ist, umsetzt,

b) während der Umsetzung gebildetes Wasser, gegebenenfalls unter leichtem Vakuum, fortlaufend entfernt,

c) nach Beendigung der Reaktion unter fortgesetztem, mechanischem Vermischen auf eine Temperatur von Umgebungstemperatur bis 40° C abkühlt und gebildetes bzw. vorhandenes Restwasser im Vakuum entfernt sowie

d) die gebildeten, pulverförmigen Metallseifen dem Reaktor entnimmt, wobei

e) während der gesamten Umsetzung einschließlich der Entfernung von Reaktions- bzw. Restwasser das Reaktionsgemisch in Form von diskreten, freifließenden Teilchen vorliegt.

Mit dem Verfahren der Erfindung lassen sich sämtliche pulverförmigen Fettsäuren mit 8 bis 34 Kohlenstoffatomen natürlichen oder synthetischen Ursprungs in die gewünschten basischen Metallseifen überführen; bevorzugt werden die gesättigten Fettsäuren eingesetzt. Besonders vorteilhaft ist das Verfahren für die Umsetzung von technischen Gemischen natürlicher, gesättigter Fettsäuren mit 14 bis 34, insbesondere 14 bis 22 Kohlenstoffatomen, die einen Schmelzpunkt oberhalb der Umsetzungstemperatur des Verfahrens der Erfindung aufweisen; typische Beispiele für derartige Fettsäuren sind Palmitin-, Stearin-, Behen- und Montansäuren sowie technische Gemische, die reich an den genannten Fettsäuren sind. Als dem Reaktionsgemisch zuzusetzende Säuren eignen sich starke bis mittelstarke Säuren mit einem pKₛ-Wert von 5 oder weniger als 5, z.B. anorganische Säuren wie Salz-, Schwefel- und Phosphorsäure sowie organische Säuren wie Ameisensäure, Essigsäure, Propionsäure oder dergleichen.

Gemäß einer weiteren vorteilhaften Ausführungsform der Erfindung steuert bzw. regelt man die Umsetzungstemperatur durch die durch das Mischwerkzeug verursachte Friktionswärme.

Als Reaktoren eignen sich sämtliche bekannten Mischwerkzeuge zur Intensivvermischung von freifließenden Teilchen.

Die Erfindung wird im folgenden anhand bevorzugter Ausführungsbeispiele näher erläutert.

Die in den folgenden Beispielen beschriebene Herstellung von basischen Metallseifen erfolgte bei Temperaturen unterhalb der Schmelzpunkte der eingesetzten Fettsäuren. Als Reaktor wurde ein Waring-Mixbecher mit einem Volumen von 1000 ml und einem variabel einstellbaren Rührwerk ( 300 bis 2000 U/min) eingesetzt.

Die eingesetzten Säuren waren Stearinsäure (technisch; ca. 1:1-Gemisch von Stearin- und Palmitinsäure; Tropfpunkt 55 °C; Zahlenmittel des Molekulargewichts ca. 270), Behensäure (technisch; ca. 90 % Behensäure, Rest Arachin- und Stearinsäure; Tropfpunkt ca. 70 °C; Zahlenmittel des Molekulargewichts ca. 340) und Myristinsäure (ca. 93 %-ig, 2 % $C_{12}$, 5 % $C_{16}$, Tropfpunkt ca. 46 °C; Zahlenmittel des Molekulargewichts ca. 228).

Beispiel 1.

Herstellung von basischem Calciumstearat. $(CaO)_{1,1} \cdot Ca(Stearat)_2$

108,0 g Stearinsäure und 31,1 g Calciumhydroxid wurden in den Mischer vorgelegt und unter Rühren auf 40-45 °C erwärmt. Nach Zugabe von 0,7 g Essigsäure (0,5 % auf Gesamtansatz) lief die Reaktion schwach exotherm unter Erwärmen auf ca. 65 °C ab. Es lagen während der Umsetzung zu keiner Zeit flüssige Säureanteile vor. Die Rührzeit betrug insgesamt 5 Minuten, wobei nach 2 Minuten bereits ein staubendes Pulver vorlag. Die Reaktion wurde unter schwachem Vakuum (700 bis 900 hPa) durchgeführt.

Man erhielt ein weißliches, sehr feines, fließfähiges Pulver mit einem Schmelzpunkt (Kofler) von 160 bis über 250 °C, einem Ca-Gehalt von 11,0 %, einer Säurezahl von 4,0 und einem Restwassergehalt von 1,8 %.

Beispiel 2.

Herstellung von Calciumbehenat (20 % basisch). $(CaO)_{0,2} \cdot Ca(Behenat)_2$

139,9 g Behensäure und 19,0 g Calciumhydroxid wurden vorgelegt und bei 60 bis 65 °C unter Rühren gemischt. Nach Zugabe von 0,8 g Essigsäure (0,5 % bezogen auf Gesamtansatz) stieg die Temperatur auf 85 °C an. Gerührt wurde insgesamt 30 Minuten; nach 2 Minuten lag bereits ein staubiges Pulver vor. Die Reaktion wurde wie im Beispiel 1 unter leichtem Vakuum durchgeführt.

Man erhielt ein weißes, lockeres Pulver mit einem Schmelzpunkt (Kofler) von 150 °C, einem Ca-Gehalt von 6,6 %, einer Säurezahl von 2,3 und einem Restfeuchtegehalt von 1,5 %.

Beispiel 3.

Herstellung von 2-basischem Bleistearat. $(PbO)_2 \cdot Pb(Stearat)_2$

101,1 g PbO (neutral) und 81,6 g Stearinsäure wurden vorgelegt und bei 45 °C vermischt. Nach Zugabe von 0,9 g Essigsäure (0,5 % auf Gesamtansatz) verlief die Reaktion schwach exotherm unter Temperaturerhöhung auf ca. 65 °C. Gelegentlich auftretende leichte Verklumpungen an der Gefäßwandung lassen sich mit einer Abstreifvorrichtung beseitigen. Es wurde 20 Minuten im leichten Vakuum gerührt.

Man erhielt ein schwach gelbliches Pulver mit einem Schmelzpunkt (Kofler) von 112 bis über 250 °C, einem Bleigehalt von 51,0 °C, einer Säurezahl von 2,6 und einem Restfeuchtegehalt von 0,2 %.

Beispiel 4.

Herstellung von basischem Calciummyristat. $(CaO)_{1,08} \cdot Ca(Myristat)_2$

114,5 g Myristinsäure und 38,9 g Calciumhydroxid wurden bei 35 bis 40 °C verrührt und mit 0,3 % Essigsäure als Katalysator versetzt. Zunächst bildete sich eine leichte Verdichtung der Masse aus, sodann ein weißes voluminöses Pulver. Während der Reaktion stieg die Temperatur auf 70 bis 80 °C. Die gesamte Rührzeit betrug 3 Minuten. Es wurde ein weißes voluminöses Pulver mit einem Ca-Gehalt von 13,6 %, einem Schmelzpunkt (Kofler) von 180 bis über 250 °C und einer Säurezahl von 1,6 erhalten.

Beispiel 5.

Herstellung von basischem Calciumbehenat. $(CaO)_{1,12} \cdot Ca(Behenat)_2$

108,8 g Behensäure und 24,8 g Calciumhydroxid wurden bei 60 bis 70 °C verrührt und mit 0,4 % Essigsäure versetzt. Die gesamte Rührzeit betrug 15 Minuten. Während der Reaktion stieg die Temperatur auf 90 °C. Man erhielt ein weißliches, feines, lockeres Pulver mit einem Ca-Gehalt von 10,1 %, einem Schmelzpunkt (Kofler) von 140 bis über 250° C und einer Säurezahl von 4,3.

Beispiel 6.

Herstellung von basischem Bleistearat. PbO . Pb-(Stearat)$_2$

100,4 g Stearinsäure und 83,0 g Bleioxid wurden bei 45° C verrührt und mit 0,3 % Essigsäure versetzt. Nach einminütigem Rühren stellte sich eine leichte Verklumpung ein. Man ließ 5 Minuten abkühlen und rührte erneut. Nach 20 Minuten lag ein schwach gelbliches Pulver mit einem Bleigehalt von 40,9 %, einem Schmelzpunkt (Kofler) von 114° C und einer Säurezahl von 2,0 vor.

Beispiel 7.

Herstellung von 2-basischem Bleistearat. (PbO)$_2$ . Pb(Stearat)$_2$

81,6 g Stearinsäure und 101,1 g PbO sowie 1 %, bezogen auf den Gesamtansatz, Ethanol wurden bei 45° C vermischt und mit 0,3 % Essigsäure versetzt. Während des Rührens bildeten sich Anbackungen an der Gefäßwandung, die abgelöst werden konnten. Nach 5 Minuten wurde das Produkt pulverig unter Erwärmung auf ca. 65° C; danach trat kein Anbacken mehr auf. Die gesamte Rührzeit betrug 10 Minuten. Man erhielt ein schwach gelbliches Pulver mit einem Bleigehalt von 51,6 %, einem Schmelzpunkt (Kofler) von 114 bis über 250° C und einer Säurezahl von 3,9.

Beispiel 8.

Herstellung von basischem Magnesiumstearat. (MgO)$_{1,2}$ . Mg(Stearat)$_2$

161,5 g Stearinsäure und 27,0 g MgO wurden bei 45° C verrührt und mit 1,5 % 50 %-iger Essigsäure versetzt. Zu Beginn des Rührens trat ein leichtes Verklumpen der Mischung auf. Nach ca. 30 Minuten lag ein gut rührbares Pulver vor. Man erhielt ein weißliches Pulver mit einem Mg-Gehalt von 8,0 %, einem Schmelzpunkt (Kofler) von 110 bis 215° C und einer Säurezahl von 5,5.

Beispiel 9.

Herstellung von basischem Zinkstearat. ZnO . Zn-(Stearat)$_2$

118,1 g Stearinsäure und 36,3 g Zinkoxid wurden bei 45° C verrührt und mit 1,5 % 30 %-iger Essigsäure versetzt. Während des Rührens bildeten sich zunächst noch Anbackungen an der Gefäßwand, die sich ablösen ließen. Nach 5 Minuten war der Ansatz gut rührbar, nach 10 Minuten lag ein staubendes, weißliches, etwas voluminöses Pulver mit einem Zinkgehalt von 18,8 %, einem Schmelzpunkt (Kofler) von 128° C und einer Säurezahl von 4,1 vor.

Beispiel 10.

Herstellung von basischem Blei/Calcium-Stearat. (MO)$_{1,8}$ . M(Stearat)$_2$; M = Ca, Pb

73,7 g Stearinsäure, 76,2 g PbO und 3,6 g Calciumhydroxid wurden bei 40 bis 45° C verrührt und mit 1 % 50 %-iger Essigsäure versetzt. Die Temperatur stieg auf ca. 70° C an. Insgesamt wurde 35 Minuten gerührt. Man erhielt ein schwach gelbliches Pulver mit einem Schmelzpunkt (Kofler) von 58 bis über 250° C, einem Bleigehalt von 46,1 %, einem Calciumgehalt von 1,2 % und einer Säurezahl von 3,1.

Beispiel 11.

Herstellung von basischem Calcium/Zink-Stearat. MO . M(Stearat)$_2$; M = Ca, Zn

145,3 g Stearinsäure, 26,6 g Calciumhydroxid und 14,6 g Zinkoxid wurden bei 40 bis 45° C miteinander vermischt und mit 1 % 50 %-iger Essigsäure versetzt. Nach zweiminütigem Rühren setzte die Reaktion ein. Innerhalb von 15 Minuten stieg die Temperatur auf ca. 75° C. Es wurde 45 Minuten gerührt. Man erhielt ein weißliches, lockeres Pulver mit einem Schmelzpunkt (Kofler) von 155 bis über 250° C, einem Calciumgehalt von 7,6 %, einem Zinkgehalt von 6,5 % und einer Säurezahl von 2,1.

Beispiel 12.

Herstellung von basischem Barium/Zink-Stearat. (MO)$_{1,12}$ . M(Stearat)$_2$; M = Ba, Zn

123,2 g Stearinsäure, 59,6 g Bariumhydroxid (eingesetzt als Bariumhydroxid-Monohydrat) und 14,8 g Zinkoxid wurden bei 40 bis 45 °C miteinander vermischt und mit 1 % 50 %-iger Essigsäure versetzt. Die Temperatur stieg im Reaktionsverlauf bis auf 65 °C an. Nach 35-minütigem Rühren lag ein weißliches, lockeres Pulver mit einem Schmelzpunkt (Kofler) von 104 bis ber 250 °C, einem Bariumgehalt von 20,5 %, einem Zinkgehalt von 6,6 % und einer Säurezahl von 2,4 vor.

Beispiel 13.

Herstellung von basischem Barium/Cadmium-Stearat. $(MO)_{1,2} \cdot M(Stearat)_2$; M = Ba, Zn

117,6 g Stearinsäure, 71,3 g Bariumhydroxid (als Monohydrat) und 14,0 g Cadmiumhydroxid wurden bei 40 bis 45 °C gemischt und mit 1 % 50 %-iger Essigsäure versetzt. Es wurde insgesamt 35 Minuten gerührt, wobei die Temperatur auf 65 °C anstieg. Man erhielt ein weißliches, lockeres, fließfähiges Pulver mit einem Schmelzpunkt (Kofler) von 106 bis über 200 °C, einem Bariumgehalt von 22,8 %, einem Cadmiumgehalt von 6,3 % und einer Säurezahl von 1,9.

## Ansprüche

1. Verfahren zur Herstellung von pulverförmigen basischen Metallseifen der Zusammensetzung gemäß Formel (I)
$(MO)_n \cdot M(RCOO)_2$
in der MO ein Oxid aus der von CaO, ZnO, MgO, BaO und PbO gebildeten Gruppe oder ein gemischtes Oxid aus der von PbO/CaO, CaO/ZnO, BaO/CdO und BaO/ZnO gebildeten Gruppe, M eines der vorgenannten Metalle, die Gruppe $RCOO^-$ der Rest einer Fettsäure mit 8 bis 34 Kohlenstoffatomen oder von Gemischen dieser Fettsäuren und n eine Zahl von 0,1 bis 2, insbesondere von 1 bis 2 bedeutet, dadurch gekennzeichnet, daß man in einem Reaktor unter mechanischem Vermischen

a) pulverförmige Fettsäuren der Formel RCOOH, in der R wie oben definiert ist, mit pulverförmigen Metalloxiden bzw. Metalloxidgemischen der Formel MO, in der M wie oben definiert ist, oder die entsprechenden pulverförmigen Metallhydroxide bzw. Metallhydroxidgemische der Formel $M(OH)_2$ in der obigen Formel (I) entsprechenden stöchiometrischen Mengen in Gegenwart von 0,1 bis 5 Gew.-% Wasser und/oder $C_1$-$C_3$-Alkanolen und/oder 0,1 bis 1 Gew.-% einer Säure mit einem $pK_s$-Wert von 5 oder weniger als 5 bei Temperaturen zwischen Umgebungstemperatur und 100 °C und einem Druck, der gleich dem oder geringer als der Umgebungsdruck ist, umsetzt,

b) während der Umsetzung gebildetes Wasser, gegebenenfalls unter leichtem Vakuum, fortlaufend entfernt,

c) nach Beendigung der Reaktion unter fortgesetztem, mechanischem Vermischen auf eine Temperatur von Umgebungstemperatur bis 40 °C abkühlt und gebildetes bzw. vorhandenes Restwasser im Vakuum entfernt sowie

d) die gebildeten, pulverförmigen Metallseifen dem Reaktor entnimmt, wobei

e) während der gesamten Umsetzung einschließlich der Entfernung von Reaktions- bzw. Restwasser das Reaktionsgemisch in Form von diskreten, freifließenden Teilchen vorliegt.

2. Verfahren nach Anspruch 1, dadurch gekennzeichnet, daß man die Umsetzungstemperatur durch die durch das Mischwerkzeug verursachte Friktionswärme steuert bzw. regelt.

3 Verfahren nach Anspruch 1 oder 2, dadurch gekennzeichnet, daß man als Fettsäuren ein technisches Gemisch natürlicher Fettsäuren mit 14 bis 34, insbesondere 14 bis 22 Kohlenstoffatomen mit einem Schmelzpunkt oberhalb der Umsetzungstemperatur einsetzt.